# EUROPEAN PATENT APPLICATION

(11) **EP 4 534 072 A1**
(43) Date of publication of application: **09.04.2025**
(21) Application number: 23202070.1
(22) Date of filing: 06.10.2023
(51) Int. Cl.: A61K 9/00, A61K 9/06, A61K 31/192, A61K 47/10, A61P 17/02, A61P 17/10, A61P 17/12

(54) **NON-AQUEOUS PHARMACEUTICAL GEL FORMULATION CONTAINING DISSOLVED ADAPALENE**

(71) Applicant: Dr. August Wolff GmbH & Co. KG Arzneimittel, 33611 Bielefeld (DE)
(72) Inventor: GILLER, Sven, 33611 Bielefeld (DE); LAUTERBACH, Andreas, 33611 Bielefeld (DE)
(74) Representative: Ter Meer Steinmeister & Partner

(57) **Abstract**

The present invention relates to a non-aqueous pharmaceutical gel composition comprising adapalene or a pharmaceutically acceptable salt thereof, at least one hydrophilic solvent, at least one hydrophilic co-solvent, and at least one thickening agent, wherein adapalene or the pharmaceutically acceptable salt thereof is dissolved within the gel composition. The non-aqueous pharmaceutical gel composition of the invention is suitable for use in the treatment of skin, particularly wherein the skin is keratinized.

## Description

The present invention relates to a non-aqueous pharmaceutical gel composition comprising adapalene or a pharmaceutically acceptable salt thereof, at least one hydrophilic solvent, at least one hydrophilic co-solvent, and at least one thickening agent, wherein adapalene or the pharmaceutically acceptable salt thereof is dissolved within the gel composition. The non-aqueous pharmaceutical gel composition of the invention is suitable for use in the treatment of skin, particularly wherein the skin is keratinized.

### Background of the invention

The epidermis constitutes the outermost of the three layers that comprise the skin, the inner layers being the dermis and hypodermis. The epidermis functions to protect the underlying tissues from environmental influences such as physical damage, infection, desiccation, UV radiation, heat loss, and to maintain homeostasis.

The epidermis is composed of four or five layers, depending on the region of skin being considered. Those layers from innermost to outermost are basal/germinal layer (*stratum basale*/*germinativum*)*,* spinous layer (*stratum spinosum*)*,* granular layer (*stratum granulosum*)*,* clear/translucent layer (*stratum lucidum,* only in palms and soles), cornified layer (*stratum corneum*)*.* The epidermis is separated from the dermis, its underlying tissue, by a basement membrane.

The epidermis consists of ninety percent keratinocytes held together by desmosomes. In the outer layers it consists of keratinized squamous corneocytes.

Keratinization is the acquisition of a horn-like character by the epithelial tissue in many parts of the body. Commonly, keratinization is distinct and pronounced in the epidermis as the outermost layer of mammal and human skin. The keratinization process starts from the keratinocytes of the basal layer *(stratum basale)* of the epidermis up to the corneocytes of the cornified layer (*stratum corneum*)*.*

In diseases of hyperkeratinization the keratinization process is altered due to marked differentiation and proliferation rates of keratinocytes. Specifically, the keratinization process is altered in diseases of the skin such as warts, acne, keratosis pilaris, and other keratinization disorders (Van Scott and Yu. Hyperkeratinization, corneocyte cohesion, and alpha hydroxy acids. J Am Acad Dermatol. 1984 Nov;11(5 Pt 1):867-79).

Active pharmaceutical ingredients with effects on differentiation and proliferation of keratinocytes are for instance retinoids such as tretinoin, acitretin, isotretinoin, etretinate, adapalene, alitretinoin, and tazarotene. Hence, retinoids are common and important ingredients in topical formulations used for cosmetic or dermatologic or medical treatment of the skin.

In particular, adapalene is an important mainstay in the treatment of acne. However, it can also be utilized to treat other conditions characterized by hyperkeratinization such as plantar warts (Gupta. Plantar warts treated with topical adapalene. Indian J Dermatol. 2011 Sep-Oct; 56(5): 513-514; R. Gupta, S. Gupta: Topical adapalene in the treatment of plantar warts; randomized comparative open trial in comparison with cryo-therapy. Indian J Dermatol (2015) 60(1):102).

For the treatment of various conditions characterized by hyperkeratinization (hyperkeratosis), particularly proliferation hyperkeratosis, the delivery of adapalene to the epidermis in high quantities is necessary.

However, distinct cornified layers of the *stratum corneum* form a barrier for penetration of substances such as active pharmaceutical ingredients into and/or across the epidermis (Trommer and Neubert. Overcoming the stratum corneum: the modulation of skin penetration. A review. Skin Pharmacol Physiol. 2006;19(2):106-21). Thus, for enhanced penetration into the epidermis active pharmaceutical ingredients need to be dissolved in the formulation and must remain dissolved in the formulation that is formed after application on the skin as the secondary and tertiary formulation whereas the secondary formulation is formed during and after application and the tertiary formulation is the residual formulation (Surber and Knie. Metamorphosis of Vehicles: Mechanisms and Opportunities. Curr Probl Dermatol. 2018;54:152-165).

Many active pharmaceutical ingredients such as adapalene are poorly soluble in several common solvents and, therefore, feature a limited penetration profile into the epidermis of skin from topically applied formulations.

Accordingly, approved pharmaceutical products used for the treatment of acne and other hyperkeratinization disorders, lack the ability to deliver adapalene to the epidermis in high quantities, without permeation of the active ingredient.

Thus, Differin^{®} gel, a commonly used aqueous gel formulation containing adapalene in a dispersed (solid) state does not allow the compound to penetrate the epidermis in sufficient concentrations. Similarly, Differin^{®} cream also contains dispersed (solid) adapalene crystals with a limited epidermal penetration rate.

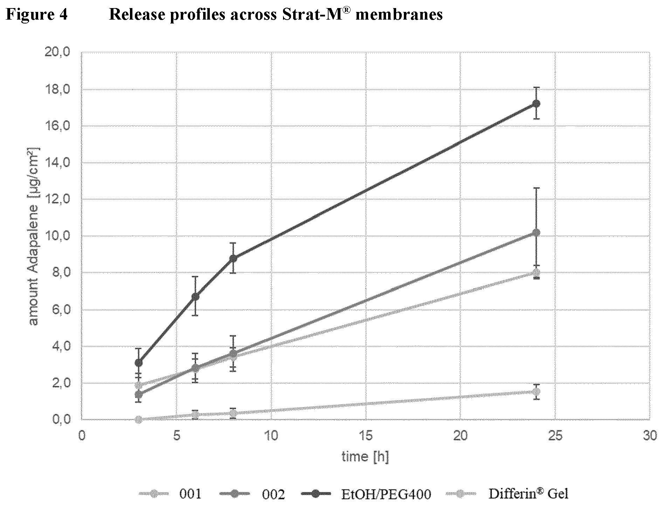

On the other hand, formulations containing adapalene in a dissolved state, such as adapalene topical solution, are liquid (alcoholic) solutions, which, when applied to a lesion, do not stick to the application area. Furthermore, when applied to the skin as a thin layer (e. g. finite dosing), upon evaporation of alcohol in the formulation, adapalene precipitates, prohibiting the compound from penetrating into the epidermis. In contrast, when the alcoholic solution is applied in a way, that prevents the alcohol from evaporating (e. g. infinite dosing, occlusive), the formulation leads to adapalene permeating across the epidermis as shown exemplary in Figure 4. Therefore, known solutions of adapalene used for the treatment of hyperkeratinization disorders are not suitable for targeted delivery of adapalene into the epidermis.

Further formulations of the active pharmaceutical ingredient adapalene are described as follows:
Verschoore et al. (Efficacy and safety of CD 271 alcoholic gels in the topical treatment of acne vulgaris. Br J Dermatol. 1991 Apr; 124(4):368-71) mentions alcoholic gels of adapalene for the topical treatment of acne vulgaris, whereas Rolland et al. (Influence of Formulation, Receptor Fluid, and Occlusion, on in Vitro Drug Release from Topical Dosage Forms, Using an Automated Flow-Through Diffusion Cell. Pharm Res 9, 82-86 (1992)) describes a solution consisting of 70% polyethylene glycol 400 and 30% ethanol, a hydroalcoholic gel consisting of 45% ethanol, an aqueous gel, an anionic oil-in-water emulsion and a nonionic oil-in-water emulsion.

WO 2006/070093 A1 (Composition comprising solubilized adapalene with cyclodextrins) describes aqueous formulations with cyclodextrins and solubilized adapalene, wherein adapalene forms an inclusion complex with cyclodextrins.

WO 2009/156675 A2 (Novel depigmenting compositions in the form of a petroleum jelly-free and elastomer-free anhydrous composition) describes anhydrous lipophilic formulations based on glyceryl behenate with a phenolic active pharmaceutical ingredient and adapalene.

Bhatia et al. (Adapalene Microemulsion for Transfollicular Drug Delivery. J Pharm Sci (2013) 102(8) 2622-2631) describes microemulsions of adapalene composed of oleic acid, tween 20, Transcutol^{®}, and water to enhance follicular penetration, whereas Trichard et al. (Novel Beads Made of Alpha-cyclodextrin and Oil for Topical Delivery of a Lipophilic Drug. Pharm Res (2008) 25(2) 435-440) describes beads of alpha-cyclodextrin with soybean oil and adapalene.

US 8,404,220 B2 (Production of adapalene gels) describes a manufacturing process to produce aqueous adapalene gels where adapalene is dispersed in the gel.

US 9,987,239 B1 (Pharmaceutical retinoid preparation for topical use) describes the combination of adapalene with a solvent-lipid complex in water.

WO 2019/08209 A1 (Topical pharmaceutical composition of adapalene and minocycline) describes formulations of adapalene and minocycline in different formulations where adapalene is dispersed therein.

WO 2022/055927 A1 (Topical compositions and methods of use) describes topical compositions containing active ingredients such as adapalene based on surfactants, viscosity enhancers, and permeation enhancers in water-based formulations.

Thus, the active pharmaceutical ingredient adapalene is most commonly suspended (dispersed) in various dosage forms such as hydrophilic gels, creams, and emulsions. These formulations are aqueous based and have a high amount of water. Further, these dosage forms exhibit non-Newtonian behavior and can be administered to skin.

Adapalene is also formulated as a solution based on polyethylene glycol and ethanol where the solution features Newtonian behavior and low adhesion properties to surfaces and the skin. Ethanol evaporates from the skin after application due to its high volatility and alters the solubility profile of adapalene in the secondary and tertiary formulation on the skin surface after topical administration due to the solvent loss.

In view of the above, the formulations of adapalene which are currently available for dermatological treatments are not suitable to deliver adapalene to the epidermis in a targeted manner and in sufficient concentrations and/or are not suitable to sustainably administer adapalene to localized diseased skin areas and lesions.

Therefore, there is still a need in the prior art for new adapalene-containing compositions having improved properties.

Accordingly, the object underlying the present invention is the provision of novel adapalene-containing compositions with improved properties, in particular for the improved treatment of conditions of the skin associated with hyperkeratinization (hyperkeratosis).

This object is achieved by the provision of the non-aqueous pharmaceutical gel composition of the present invention.

### Summary of the invention

The present invention relates to a non-aqueous pharmaceutical gel composition comprising
(a) adapalene or a pharmaceutically acceptable salt thereof;
(b) at least one hydrophilic solvent selected from the group consisting of polyethylene glycol, propylene glycol, dipropylene glycol, butylene glycol, hexylene glycol, 1,2,6-hexanetriol, and polypropylene glycol;
(c) at least one hydrophilic co-solvent selected from the group consisting of diethylene glycol monoethyl ether and dimethyl isosorbide;
(d) at least one thickening agent selected from the group consisting of polyvinyl pyrrolidone (PVP), hydroxypropyl cellulose (HPC) and Polyacrylate Crosspolymer-6.

Adapalene or the pharmaceutically acceptable salt thereof preferably is dissolved within the gel composition. The present invention also relates to the non-aqueous pharmaceutical gel composition for use in the treatment of skin, particularly keratinized skin. The present invention further relates to the non-aqueous pharmaceutical gel composition for use in the treatment of acne, warts, hyperkeratosis, keratoses, folliculitis, and ichthyosis.

It was surprisingly found that the non-aqueous pharmaceutical gel composition according to an embodiment of the present invention comprises dissolved adapalene, and that adapalene remains dissolved during storage and after application. Further surprisingly, the non-aqueous pharmaceutical gel composition according to the present invention provides for enhanced and targeted epidermal penetration of adapalene and is, therefore, useful for application to skin and for treatment of skin, particularly keratinized skin. In that respect, it was particularly surprising that the gel composition according to the present invention is able to overcome the disadvantages of currently available adapalene formulations. Thus, the gel composition according to the present invention is advantageously suitable to deliver adapalene into the epidermis, while commercially available Differin^{®} gel is not suitable to deliver adapalene to the target site, i.e. the epidermis, in sufficient concentrations, and while topical alcoholic solutions of adapalene, depending on the mode of application, either do not penetrate or penetrate not only into but also across the epidermis and are, therefore, not suitable for targeted delivery of adapalene to the epidermis.

It was further surprisingly found that the non-aqueous pharmaceutical gel composition according to the present invention exhibits non-Newtonian behavior and adhesive properties for local skin application and treatment.

Thus, the non-aqueous pharmaceutical gel composition of the present invention comprising adapalene or a pharmaceutically acceptable salt thereof surprisingly allows to deliver adapalene into the epidermis in sufficient and high concentrations and is suitable for administration of adapalene to localized diseased skin areas and lesions.

### Brief description of the figures

Figure 1 shows the solubility of adapalene in binary solvent mixtures based on polyethylene glycol 400. In Figure 1A a binary solvent mixture of polyethylene glycol 400 and diethylene glycol monoethyl ether is used and in Figure 1B a binary solvent mixture of polyethylene glycol 400 and dimethyl isosorbide is used.
Figure 2 shows micrographs of dissolved adapalene in gels (Fig. 2A, Formulation 003; Fig. 2B, Formulation 004) compared to Differin^{®} gel (Fig. 2C) and Differin^{®} cream (Fig. 2D).
Figure 3 shows the viscosity and adhesion forces of adapalene gels. Figure 3A shows the viscosity profile of Formulation 003, Figure 3B shows the viscosity profile of Formulation 004, Figure 3C shows the adhesion force profile of Formulation 003, and Figure 3D shows the adhesion force profile of Formulation 004.
Figure 4 shows release profiles of Formulation 001 ("001"), Formulation 002 ("002"), adapalene topical solution composed of polyethylene glycol 400 and ethanol ("EtOH/PEG400") and Differin^{®} gel ("Differin^{®} Gel") across Strat-M^{®} membranes.
Figure 5 shows skin penetration profiles of Differin^{®} gel (Fig. 5A), Formulation 003 (Fig. 5B) and Formulation 004 (Fig. 5C).

### Detailed description of the invention

To administer a topical formulation on localized diseased skin areas and on lesions a formulation has preferentially a high viscosity and adhesive properties.

Preferentially, gels feature the potential to deliver active pharmaceutical ingredients to skin.

Gels are semi-solid and single-phase formulations composed of active pharmaceutical ingredients, solvents or dispersion mediums, and gelling agents and/or thickening agents.

Specifically, hydrophilic gels contain hydrophilic liquids usually such as water, glycerol or propylene glycol and gelling agents like poloxamers, starch, cellulose derivatives, carbomers or magnesium-aluminum silicates, whereas lipophilic gels are based on paraffin and fatty oils gelled with polyethylene, colloidal silica or aluminum or zinc soaps.

Compared to that, creams are homogeneous formulations with a lipophilic and aqueous phase where in lipophilic creams the outer phase is lipophilic and in hydrophilic creams the outer phase is based on water.

Ointments are semi-solid and based on a single phase with dispersed solids or liquids where there are hydrophobic ointments composed of paraffins, fatty oils, fats, glycerides, waxes, and polyalkylsiloxanes, water-emulsifying ointments with water-in-oil emulsifiers or oil-in-water-emulsifiers, and hydrophilic ointments based on liquid and solid polyethylene glycols.

Pastes are semi-solid formulations with a high solid material content.

Poultices are semi-solid formulations with a hydrophilic heat-retentive basis (Ph. Eur. 07/2021:0132).

According to the present invention, adapalene is formulated in a non-aqueous pharmaceutical gel composition. The inventive formulations in the form of a non-aqueous pharmaceutical gel composition surprisingly enable the dissolution of adapalene in a gel formulation. Such gel formulation may advantageously exhibit a dynamic viscosity of more than 0.3 Pa·s at 100 s⁻¹ and an adhesion of more than 0.7 N to administer the formulations locally to skin with a subsequent enhanced and targeted epidermal penetration.

The non-aqueous pharmaceutical gel composition according to the present invention comprises
(a) adapalene or a pharmaceutically acceptable salt thereof;
(b) at least one hydrophilic solvent selected from the group consisting of polyethylene glycol, propylene glycol, dipropylene glycol, butylene glycol, hexylene glycol, 1,2,6-hexanetriol, and polypropylene glycol;
(c) at least one hydrophilic co-solvent selected from the group consisting of diethylene glycol monoethyl ether and dimethyl isosorbide;
(d) at least one thickening agent selected from the group consisting of polyvinyl pyrrolidone (PVP), hydroxypropyl cellulose (HPC) and Polyacrylate Crosspolymer-6.

Adapalene or the pharmaceutically acceptable salt thereof is dissolved within the gel composition.

According to the invention, adapalene can be present in the form of the free acid or as a pharmaceutically acceptable salt thereof.

The pharmaceutically acceptable salt of adapalene can be a salt of adapalene with an alkaline or alkaline earth metal, preferably a salt of adapalene with sodium or potassium.

According to the present invention, adapalene or the pharmaceutically acceptable salt thereof is dissolved within the gel composition. The term "dissolved" means herein that adapalene is molecularly dispersed in the composition. In other words, from a technical perspective, in the "dissolved" state no adapalene crystals are observed via polarized light microscopy, e.g. at a magnification of 200x and a resolution of 1 µm. Adapalene is in a dissolved state at least at ambient conditions corresponding to climate zones I-IV. Furthermore, "dissolved adapalene" herein does not mean that adapalene is (primarily) dissolved as a complex such as an inclusion complex (e.g. with cyclodextrins). In other words, adapalene according to the invention is not primarily present as a complex, particularly not as an inclusion complex with cyclodextrins.

In a preferred embodiment of the invention, the concentration of adapalene is in a range of 0.01 - 0.3% (w/w), preferably 0.05 - 0.2% (w/w), preferably 0.1% (w/w).

The solvent of the non-aqueous pharmaceutical gel composition according to the present invention is a component that can dissolve adapalene, wherein the solvent for adapalene is not water due to poor aqueous solubility of adapalene.

The solvent of the non-aqueous pharmaceutical gel composition of the present invention is at least one hydrophilic solvent selected from the group consisting of polyethylene glycol, propylene glycol, dipropylene glycol, butylene glycol, hexylene glycol, 1,2,6-hexanetriol, and polypropylene glycol. In particular, the solvent of the non-aqueous pharmaceutical gel composition of the present invention is at least one glycol, particularly a glycol selected from the group consisting of polyethylene glycol, propylene glycol, dipropylene glycol, butylene glycol, hexylene glycol, and polypropylene glycol.

The polyethylene glycol is preferably a polyethylene glycol which is liquid at room temperature, preferably having an average molecular mass of 100, 200, 300, 400, or 600.

In a preferred embodiment of the invention, the hydrophilic solvent is polyethylene glycol, preferably polyethylene glycol having a relative molecular mass of 100 - 600, more preferably polyethylene glycol having a relative molecular mass of 400.

In a preferred embodiment of the invention, the concentration of the hydrophilic solvent is in a range of 45 - 98% (w/w), preferably 70 - 90% (w/w), more preferably 80 - 85% (w/w).

The co-solvent of the non-aqueous pharmaceutical gel composition according to the present invention is a component that facilitates the dissolution of adapalene in the solvent wherein the co-solvent for adapalene is not a surfactant and/or a cyclodextrin.

According to the invention, the co-solvent is a hydrophilic co-solvent. Hydrophilic co-solvents are components that are soluble in and/or miscible with water. Suitable hydrophilic co-solvents of the gel composition of the invention comprise compounds based on ether functions.

The co-solvent of the non-aqueous pharmaceutical gel composition of the present invention is at least one hydrophilic co-solvent selected from the group consisting of diethylene glycol monoethyl ether and dimethyl isosorbide.

In a preferred embodiment of the invention, the hydrophilic co-solvent is diethylene glycol monoethyl ether. In another preferred embodiment of the invention, the hydrophilic co-solvent is dimethyl isosorbide.

In a preferred embodiment of the invention, the concentration of the hydrophilic co-solvent is in a range of 1 - 50% (w/w), preferably 5 - 30% (w/w), more preferably 8.5 - 9.5% (w/w).

The thickening agent of the non-aqueous pharmaceutical gel composition according to the present invention is a component that enhances the viscosity of the composition.

According to the invention, the thickening agent is a thickening agent compatible with the solvent and the co-solvent. Thus, the thickening agent of the composition of the invention is soluble in the mixture of the solvent and co-solvent and forms a transparent gel. Accordingly, the thickening agent of the composition of the invention is a component that is dissolved in the composition and that forms a transparent composition with an enhanced viscosity.

The thickening agent of the non-aqueous pharmaceutical gel composition of the present invention is at least one thickening agent selected from the group consisting of polyvinyl pyrrolidone (PVP), hydroxypropyl cellulose (HPC) and Polyacrylate Crosspolymer-6 (currently available under the tradename SepineoTM PHD100).

PVP is also termed povidone. Suitable povidones exhibit a K-value of 85 - 95 and a mean molecular weight of 900000 - 1500000 g/mol.

Suitable hydroxypropyl cellulose exhibits an average molecular weight of 800000 - 1200000 Da.

In a preferred embodiment of the invention, the thickening agent is polyvinyl pyrrolidone, preferably povidone K85-95, and more preferably povidone K90. In another preferred embodiment of the invention, the thickening agent is hydroxypropyl cellulose.

In a preferred embodiment of the invention, the concentration of the thickening agent is in a range of 0.5 - 15% (w/w), preferably 4 - 12% (w/w), more preferably 8% (w/w).

In a preferred embodiment of the invention, the non-aqueous pharmaceutical gel composition further comprises (e) optionally at least one pharmaceutical stabilizer. The optional at least one pharmaceutical stabilizer is at least one preservative and/or at least one antioxidant.

The pharmaceutical stabilizer of the non-aqueous pharmaceutical gel composition of the present invention is a preservative selected from the group consisting of phenoxyethanol, benzyl alcohol, methylparaben, propylparaben and ethylparaben, or an antioxidant selected from the group consisting of butylated hydroxy toluene, butylated hydroxy anisole, ascorbic acid, propyl gallate, sodium sulfite, tocopherol and ascorbyl palmitate.

In a preferred embodiment of the invention, the pharmaceutical stabilizer is phenoxyethanol.

In a preferred embodiment of the invention, the concentration of the pharmaceutical stabilizer is in a range of 0.01 - 2% (w/w), preferably 0.05 - 1.5% (w/w), more preferably 0.5 - 1% (w/w).

The non-aqueous pharmaceutical gel composition according to the invention has a dynamic viscosity of more than 0.3 Pa s at a shear rate of 100 s⁻¹, preferably more than 0.5 Pa s at a shear rate of 100 s⁻¹, more preferably more than 1 Pa s at a shear rate of 100 s⁻¹, even more preferably more than 5 Pa s at a shear rate of 100 s⁻¹, and up to 45 Pa s at a shear rate of 100 s⁻¹, preferably up to 35 Pa s at a shear rate of 100 s⁻¹, more preferably up to 25 Pa s at a shear rate of 100 s⁻¹, as determined via a rotational rheometer equipped with a cone plate measurement geometry at 25°C.

The non-aqueous pharmaceutical gel composition according to the invention has an absolute adhesion force of more than 0.7 N, preferably more than 1 N, more preferably more than 2 N, even more preferably more than 10 N determined via a vertical force measurement with a rheometer equipped with a cone plate measurement geometry at 32°C.

The compositions according to the present invention are non-aqueous and/or non-alcoholic gel compositions, particularly non-aqueous and/or non-ethanolic and/or non-isopropanolic gel compositions. Thus, water and/or ethanol and/or isopropanol is/are not actively added as single component(s) to the formulations.

In one embodiment, the pharmaceutical gel composition according to the present invention is a non-aqueous composition. This means that the pharmaceutical gel composition contains less than 10% (w/w) water, preferably less than 5% (w/w) water, more preferably less than 2% (w/w) water. In a preferred embodiment, the pharmaceutical gel composition contains substantially no water. In other words, the pharmaceutical gel composition contains no water apart from inevitable traces thereof.

The pharmaceutical gel composition according to the present invention is a non-alcoholic composition (in particular, non-ethanolic and/or non-isopropanolic compositions). This means that the pharmaceutical gel composition contains less than 2% (w/w) ethanol and/or isopropanol, preferably less than 1% (w/w) ethanol and/or isopropanol, more preferably less than 0.5% (w/w) ethanol and/or isopropanol. In a preferred embodiment, the pharmaceutical gel composition contains substantially no ethanol and/or isopropanol. In other words, the pharmaceutical gel composition contains no ethanol and/or isopropanol apart from inevitable traces thereof.

In a preferred embodiment of the invention, the non-aqueous pharmaceutical gel composition comprises 0.1% (w/w) adapalene, 45 - 94.9% (w/w) polyethylene glycol having a relative molecular mass of 100 - 600, 1 - 50% (w/w) diethylene glycol monoethyl ether, 4 - 12% (w/w) povidone K90, and optionally a pharmaceutical stabilizer.

In a particularly preferred embodiment of the invention, the non-aqueous pharmaceutical gel composition is composed of 0.1% (w/w) adapalene, diethylene glycol monoethyl ether, povidone K 90, phenoxyethanol, and polyethylene glycol 400. Adapalene is dissolved in the formulation. The formulation e.g. has a viscosity of 5,87 Pa s at a shear rate of 100 s⁻¹ and an adhesion force of 11,23 N.

In another preferred embodiment of the invention, the non-aqueous pharmaceutical gel composition comprises 0.1% (w/w) adapalene, 45 - 94.9% (w/w) polyethylene glycol having a relative molecular mass of 100 - 600, 1 - 50% (w/w) dimethyl isosorbide, 4 - 12% (w/w) povidone K90, and optionally a pharmaceutical stabilizer.

In another particularly preferred embodiment of the invention, the non-aqueous pharmaceutical gel composition is composed of 0.1% (w/w) adapalene, dimethyl isosorbide, povidone K 90, phenoxyethanol, and polyethylene glycol 400. Adapalene is dissolved in the formulation. The formulation e.g. has a viscosity of 6,85 Pa s at a shear rate of 100 s⁻¹ and an adhesion force of 13,01 N.

The non-aqueous pharmaceutical gel compositions of the present invention are manufactured by (i) mixing the hydrophilic solvent, the hydrophilic co-solvent, and optionally the stabilizer; (ii) dissolving adapalene in the mixture obtained in step (i); and (iii) dissolving the thickening agent in the mixture obtained in step (ii) to obtain a gel.

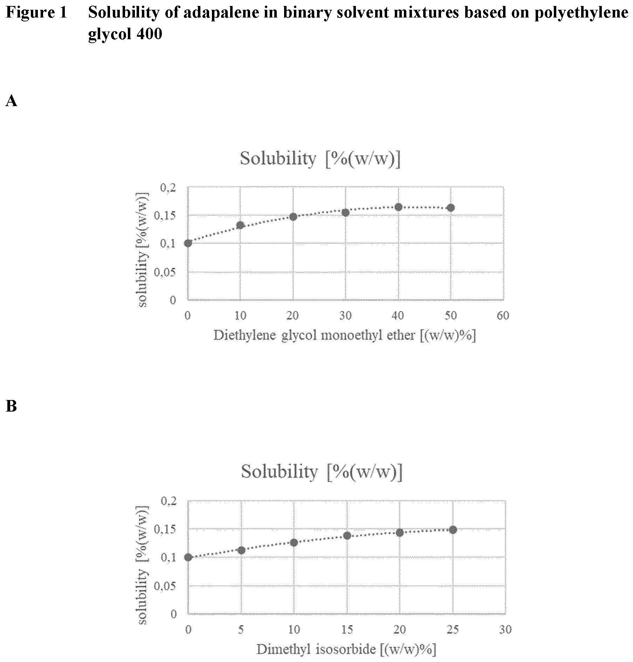

Unexpectedly, the active pharmaceutical ingredient adapalene could be dissolved in a mixture of a hydrophilic solvent and a hydrophilic co-solvent (cf. Figure 1). This was particularly surprising in view of the known general poor solubility of adapalene.

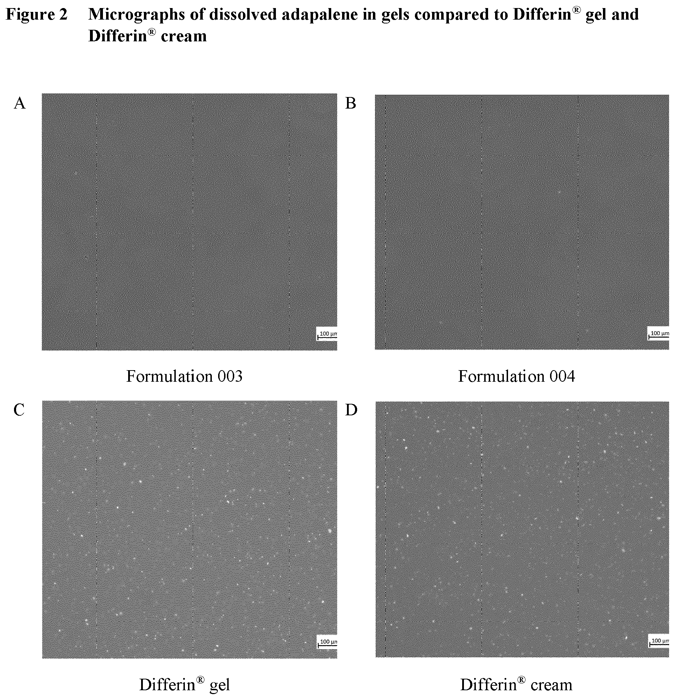

The composition of dissolved adapalene could surprisingly be formulated as a gel with dissolved adapalene. Thus, the non-aqueous pharmaceutical gel composition according to the invention comprises homogenously dissolved adapalene (cf. Fig. 2A, Formulation 003; Fig. 2B, Formulation 004). In contrast thereto, the commercially available adapalene formulations Differin^{®} gel (cf. Fig. 2C) and Differin^{®} cream (cf. Fig. 2D) comprise adapalene in a dispersed (solid) state.

Thus, the non-aqueous pharmaceutical gel composition according to the present invention comprises dissolved adapalene, and the adapalene remains dissolved during storage and after application.

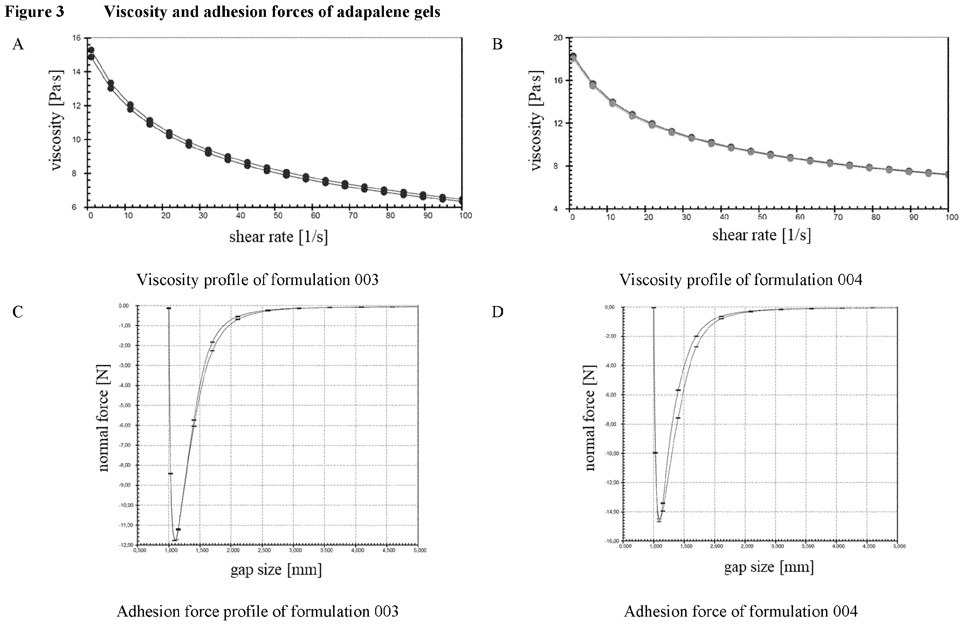

The employed thickening agents are dissolved in the formulations and form transparent gels. By using a thickening agent in the composition, the non-aqueous pharmaceutical gel composition according to the invention exhibits a viscosity of more than 1 Pa·s at a shear rate of 100 s⁻¹ (cf. Fig. 3A, Formulation 003; Fig. 3B, Formulation 004), and an absolute adhesion force of more than 2 N (cf. Fig. 3C, Formulation 003; Fig. 3D, Formulation 004).

Therefore, the non-aqueous pharmaceutical gel composition according to the invention enables targeted administration to local sites, in particular lesions of the skin and an enhanced and targeted epidermal penetration and is, therefore, useful for application to skin and for treatment of skin, particularly keratinized skin. In particular, the non-aqueous pharmaceutical gel composition according to the invention is suitable to deliver adapalene to the target site, i.e. the epidermis, in sufficient and high concentrations and is suitable for administration of adapalene to localized diseased skin areas and lesions.

The non-aqueous pharmaceutical gel composition according to the invention has several advantages over presently commercially available formulations of adapalene such as Differin^{®} gel, Differin^{®} cream and adapalene topical (ethanolic) solution. In particular, the gels according to the invention are stable during storage and feature a constant assay of adapalene, a stable viscosity, a stable adhesion, a clear macroscopic appearance, and an isotropic microscopic appearance.

Particularly advantageously, adapalene remains dissolved in the non-aqueous pharmaceutical gel composition of the invention during storage as well as after application to surfaces such as skin.

Further advantageously, the non-aqueous pharmaceutical gel composition according to the present invention exhibits non-Newtonian behavior and adhesive properties for local skin application and treatment.

Therefore, the non-aqueous pharmaceutical gel composition of the invention is particularly useful for application to skin and for treatment of skin.

In one embodiment, the non-aqueous pharmaceutical gel composition according to the invention is for use in the treatment of skin.

In a preferred embodiment, the non-aqueous pharmaceutical gel composition according to the invention is for use in the treatment of skin, wherein the skin is keratinized.

Accordingly, the non-aqueous pharmaceutical gel composition of the invention is particularly useful for application to skin and for treatment of skin, wherein the skin is keratinized such as in the case of acne, warts, hyperkeratosis, keratoses, folliculitis, and ichthyosis.

In a preferred embodiment, the non-aqueous pharmaceutical gel composition according to the invention is for use in the treatment of skin, wherein the treatment of skin comprises the treatment of acne, warts, hyperkeratosis, keratoses, folliculitis, and ichthyosis.

The invention is further illustrated by the following non-limiting examples.

### Examples

### Example 1

Four gel formulations were prepared via mixing of polyethylene glycol 400 with diethylene glycol monoethyl ether or dimethyl isosorbide and phenoxyethanol. Adapalene and povidone K 90 were dissolved in the mixture.

| **Formulation** | **001** | **002** | **003** | **004** |
|---|---|---|---|---|
| Adapalene | 0.1 | 0.1 | 0.1 | 0.1 |
| Diethylene glycol monoethyl ether | 8.6 | - | 8.6 | - |
| Dimethyl isosorbide | - | 9.1 | - | 9.1 |
| Povidone K 90 | 8 | 8 | 8 | 8 |
| Polyethylene glycol 400 | 83 | 82.5 | 82.8 | 82.3 |
| Phenoxyethanol | 0.3 | 0.3 | 0.5 | 0.5 |

The solubility of adapalene in a binary solvent mixture of polyethylene glycol 400 and diethylene glycol monoethyl ether is shown in Figure 1A and the solubility of adapalene in a binary solvent mixture of polyethylene glycol 400 and dimethyl isosorbide is shown in Figure 1B.

Figure 2 shows micrographs of dissolved adapalene in gels (Fig. 2A, Formulation 003; Fig. 2B, Formulation 004) compared to Differin^{®} gel (Fig. 2C) and Differin^{®} cream (Fig. 2D).

The homogeneous and transparent gel Formulations 003 and 004 were subjected to a stability study at 25°C and 40°C for 24 weeks.

| Formulati on | Paramete r | Temperat ure | Start | 4 weeks | 8 weeks | 12 weeks | 24 weeks |
|---|---|---|---|---|---|---|---|
| 003 | Appearan ce | 25°C | transp arent | transpare nt | transpare nt | transpare nt | transpare nt |
| | | 40°C | | transpare nt | transpare nt | transpare nt | transpare nt |
| | Microsco py | 25°C | no crysta ls | no crystals | no crystals | no crystals | no crystals |
| | | 40°C | | no crystals | no crystals | no crystals | no crystals |
| | Viscosity (at 100 s⁻¹) [Pa·s] | 25°C | 6.78 | 6.60 | 6.41 | 6.43 | 6.48 |
| | | 40°C | | 6.38 | 6.38 | 6.43 | 6.44 |
| | Adhesion force [N] | 25°C | 11.75 | 12.16 | 11.01 | 10.66 | 13.07 |
| | | 40°C | | 10.23 | 11.45 | 11.51 | 12.77 |
| | Assay [mg/g] | 25°C | 0.97 | 0.97 | 0.96 | 0.95 | 0.95 |
| | | 40°C | | 0.95 | 0.94 | 0.95 | 0.95 |
| 004 | Appearan ce | 25°C | transp arent | transpare nt | transpare nt | transpare nt | transpare nt |
| | | 40°C | | transpare nt | transpare nt | transpare nt | transpare nt |
| | Microsco py | 25°C | no crysta ls | no crystals | no crystals | no crystals | no crystals |
| | | 40°C | | no crystals | no crystals | no crystals | no crystals |
| | Viscosity (at 100 s⁻¹) [Pa·s] | 25°C | 7.70 | 7.45 | 7.06 | 7.24 | 7.33 |
| | | 40°C | | 7.15 | 7.03 | 7.06 | 6.95 |
| | Adhesion force [N] | 25°C | 14.58 | 13.15 | 11.83 | 12.80 | 13.25 |
| | | 40°C | | 13.72 | 12.58 | 12.97 | 12.88 |
| | Assay [mg/g] | 25°C | 0.99 | 0.99 | 0.96 | 0.95 | 0.96 |
| | | 40°C | | 0.97 | 0.95 | 0.95 | 0.96 |

The parameters appearance, microscopy, viscosity (at 100 s⁻¹, 25°C), adhesion force (at 32°C), and assay remained stable over the storage time.

The viscosity and adhesion forces of adapalene gels are shown in Figure 3. Figure 3A shows the viscosity profile of Formulation 003, Figure 3B shows the viscosity profile of Formulation 004, Figure 3C shows the adhesion force profile of Formulation 003, and Figure 3D shows the adhesion force profile of Formulation 004.

### Example 2

Ten different formulations with dissolved adapalene were prepared and subjected to microscopical analysis as well as viscosity and adhesion force determinations.

| **Formulation** | **005** | **006** | **007** | **008** | **009** | **010** |
|---|---|---|---|---|---|---|
| Adapalene | 0.1 | 0.1 | 0.1 | 0.01 | 0.3 | 0.05 |
| Diethylene glycol monoethyl ether | 50 | | 8.6 | | | 1 |
| Dimethyl isosorbide | | 9.1 | | 1 | 50 | |
| Povidone K 90 | 3.4 | 15 | | | | |
| Hydroxypropylcellulose 850000 | | | 1 | 1 | | |
| Hydroxypropylcellulose 1150000 | | | | | 0.5 | 0.5 |
| Polyethylene glycol 200 | | | | 97.99 | | |
| Polyethylene glycol 300 | | 74.8 | | | 48.7 | |
| Polyethylene glycol 400 | | | 89.8 | | | 97.95 |
| Polyethylene glycol 600 | 45 | | | | | |
| Phenoxyethanol | | | 0.5 | | 0.5 | |
| Benzyl alcohol | 1 | 1 | | | | |
| Butylated hydroxytoluene | 0.5 | | | | | 0.5 |
| | | | | | | |

| **Formulation** | **011** | **012** | **013** | **014** | | |
|---|---|---|---|---|---|---|
| Adapalene | 0.1 | 0.1 | 0.1 | 0.1 | | |
| Diethylene glycol monoethyl ether | 5 | 8.6 | | 4 | | |
| Dimethyl isosorbide | 5 | | 9.1 | 4 | | |
| Povidone K 90 | 8 | 1 | | 1 | | |
| Hydroxypropylcellulose 850000 | | 0.5 | | 0.5 | | |
| Hydroxypropylcellulose 1150000 | | | 1 | | | |
| Polyethylene glycol 200 | | | 44.6 | 30.1 | | |
| Polyethylene glycol 300 | 81.4 | | 44.7 | 30.1 | | |
| Polyethylene glycol 400 | | 88.8 | | 30.2 | | |
| Phenoxyethanol | 0.5 | | | | | |
| Benzyl alcohol | | 1 | | | | |
| Butylated hydroxytoluene | | | 0.5 | | | |

The homogeneous and transparent formulations were analyzed via microscopy, viscosity and adhesion force determination.

| Formulation | Microscopy | Viscosity (at 100 s⁻¹) [Pa·s] | Adhesion force [N] |
|---|---|---|---|
| 005 | transparent, no crystals | 0.35 | 0.81 |
| 006 | transparent, no crystals | 23.23 | 47.73 |
| 007 | transparent, no crystals | 1.03 | 3.42 |
| 008 | transparent, no crystals | 1.08 | 3.87 |
| 009 | transparent, no crystals | 0.44 | 1.22 |
| 010 | transparent, no crystals | 0.40 | 0.78 |
| 011 | transparent, no crystals | 6.13 | 11.47 |
| 012 | transparent, no crystals | 1.04 | 2.65 |
| 013 | transparent, no crystals | 1.41 | 4.96 |
| 014 | transparent, no crystals | 0.94 | 2.47 |

All formulations were transparent and featured no crystals, the viscosity (at 100 s⁻¹, 25°C) was between 0.35 Pa·s and 23.23 Pa·s and the adhesion force (32°C) was between 0.78 N and 47.73 N.

### Example 3

Strat-M^{®} synthetic membrane may act as an indicator for permeability of substances across skin (Haq et al. Int J Pharm (2018) 547(1-2) 432-437). It can be introduced into Franz cells to study the amount that is delivered across the membrane over time by the investigated formulation.

The homogeneous and transparent gels of Formulations 001 and 002, a solution formulation of adapalene based on polyethylene glycol 400 and ethanol and the formulation Differin^{®} gel were subjected to drug release analysis over Strat-M^{®} membrane.

The formulation Differin^{®} gel features a low release rate across a Strat-M^{®} membrane in a Franz cell system which corresponds to a distinct superficial localization of the active pharmaceutical ingredient on the skin.

Compared to that, the solution formulation of adapalene based on polyethylene glycol 400 and ethanol shows a distinct release rate across a Strat-M^{®} membrane in a Franz cell system which corresponds to a pronounced permeation through the skin.

Ethanol is volatile and a known enhancer that disrupts the barrier of the skin.

Surprisingly, the formulations according to the invention based on hydrophilic solvents and hydrophilic co-solvents (Formulation 001, and Formulation 002, exhibit a moderate release across the Strat-M^{®} membrane with a magnitude between Differin^{®} gel and the ethanolic solution.

This release behavior substantiates unexpectedly an accumulation of the active pharmaceutical ingredient in the skin and epidermis as shown in Figure 4 whereas Differin^{®} gel indicates a superficial localization and the solution of polyethylene glycol 400 and ethanol a higher permeation rate.

### Example 4

Porcine ear skin resembles human skin and can therefore be used as a model to study the penetration rate of substances into and across the skin from different formulations (Jacobi et al. Skin Res Technol (2007) 13(1) 19-24). Micrographs of penetrated fluorescent substances from sliced skin biopsies can be studied to assess the skin penetration rate via fluorescence microscopy.

Differin^{®} gel and Formulations 003 and 004 were subjected to a skin penetration analysis study where the skin penetration profile of adapalene was investigated in porcine skin from Franz cells.

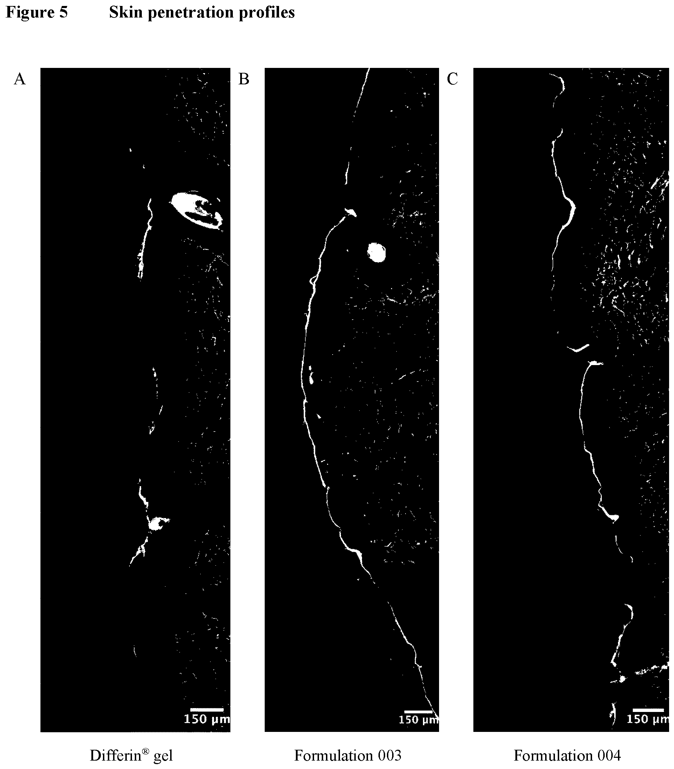

500 mg/cm² formulation was applied and occluded under parafilm at 32°C for 6 h. Samples were snap-frozen and sliced after 6 hours to analyze the fluorescence signals of adapalene via fluorescence microscopy
Micrographs show locally distinct fluorescence signals of adapalene from Differin^{®} gel (Fig. 5A) whereas the signals of adapalene from Formulation 003 (Fig. 5B) and from Formulation 004 (Fig. 5C) are homogeneously distributed within the upper skin layers (the epidermal layers are on the left side, the dermis is on the right side each in Fig. 5A-5C). Formulations 003 and 004 enable a more pronounced skin penetration of adapalene.

## Claims

1. A non-aqueous pharmaceutical gel composition comprising
(a) adapalene or a pharmaceutically acceptable salt thereof;
(b) at least one hydrophilic solvent selected from the group consisting of polyethylene glycol, propylene glycol, dipropylene glycol, butylene glycol, hexylene glycol, 1,2,6-hexanetriol, and polypropylene glycol;
(c) at least one hydrophilic co-solvent selected from the group consisting of diethylene glycol monoethyl ether and dimethyl isosorbide;
(d) at least one thickening agent selected from the group consisting of polyvinyl pyrrolidone (PVP) and hydroxypropyl cellulose (HPC),
wherein adapalene or the pharmaceutically acceptable salt thereof is dissolved within the gel composition.

2. The non-aqueous pharmaceutical gel composition according to claim 1, wherein the concentration of adapalene or the pharmaceutically acceptable salt thereof is in a range of 0.01 - 0.3% (w/w).

3. The non-aqueous pharmaceutical gel composition according to claim 1 or claim 2, wherein the hydrophilic solvent is polyethylene glycol, preferably polyethylene glycol having a relative molecular mass of 100 - 600.

4. The non-aqueous pharmaceutical gel composition according to any one of claims 1-3, wherein the concentration of the hydrophilic solvent is in a range of 45 - 98% (w/w).

5. The non-aqueous pharmaceutical gel composition according to any one of claims 1-4, wherein the hydrophilic co-solvent is diethylene glycol monoethyl ether.

6. The non-aqueous pharmaceutical gel composition according to any one of claims 1-5, wherein the concentration of the hydrophilic co-solvent is in a range of 1 - 50% (w/w).

7. The non-aqueous pharmaceutical gel composition according to any one of claims 1-6, wherein the thickening agent is polyvinyl pyrrolidone, preferably povidone K85-95, and more preferably povidone K90.

8. The non-aqueous pharmaceutical gel composition according to any one of claims 1-7, wherein the concentration of the thickening agent is in a range of 0.5 - 15% (w/w).

9. The non-aqueous pharmaceutical gel composition according to any one of claims 1-8, further comprising (e) at least one pharmaceutical stabilizer.

10. The non-aqueous pharmaceutical gel composition according to any one of claims 1-9, wherein the dynamic viscosity of the composition is more than 0.3 Pa s at a shear rate of 100 s⁻¹.

11. The non-aqueous pharmaceutical gel composition according to any one of claims 1-10, wherein the absolute adhesion force of the composition is more than 0.7 N.

12. The non-aqueous pharmaceutical gel composition according to any one of claims 1-11, comprising 0.1% (w/w) adapalene, 45 - 94.9% (w/w) polyethylene glycol having a relative molecular mass of 100 - 600, 1 - 50% (w/w) diethylene glycol monoethyl ether, 4 - 12% (w/w) povidone K90, and optionally a pharmaceutical stabilizer.

13. The non-aqueous pharmaceutical gel composition according to any one of claims 1-12 for use in the treatment of skin.

14. The non-aqueous pharmaceutical gel composition for use according to claim 13, wherein the skin is keratinized.

15. The non-aqueous pharmaceutical gel composition for use according to claim 13 and/or claim 14, wherein the treatment of skin comprises the treatment of acne, warts, hyperkeratosis, keratoses, folliculitis, and ichthyosis.
